# EUROPEAN PATENT SPECIFICATION

(11) **EP 3 200 880 B1**
(45) Date of publication and mention of the grant of the patent: **19.05.2021**
(21) Application number: 15781001.1
(22) Date of filing: 28.09.2015
(51) Int. Cl.: A61Q 5/06, A61Q 5/12, A61K 8/73, A61K 8/81, A61K 8/86, A61K 8/06

(54) **LOW VISCOSITY CELLULOSICS IN HAIR CARE COMPOSITIONS**
CELLULOSEN VON NIEDRIGER VISKOSITÄT IN HAARPFLEGEPRODUKTEN
CELLULOSE À VISCOSITÉ BASSE POUR LES COMPOSITIONS DE SOIN DE CHEVEUX

(30) Priority: 30.09.2014 US 201462057327 P
(43) Date of publication of application: 09.08.2017
(73) Proprietor: Rohm and Haas Company, Philadelphia, PA 19106 (US); Dow Global Technologies Llc, Midland, MI 48674 (US)
(72) Inventor: GREYSON, Andrea, Blue Bell Pennsylvania 19422 (US); ZHANG, Xiaodong, Belle Mead New Jersey 08502 (US)
(74) Representative: Houghton, Mark Phillip
(86) International application number: PCT/US2015/052615
(87) International publication number: WO 2016/053857

(56) References cited:
- WO-A1-2013/048779
- US-A1- 2008 260 836
- DOW CHEMICALS: "Methocel cellulose ethers - Technical handbook", INTERNET CITATION, 1 September 2002 (2002-09-01), pages 1-29, XP002629689, Retrieved from the Internet: URL:http://www.dow.com/dowwolff/en/pdfs/19 2-01062.pdf [retrieved on 2011-03-29]
- ANONYMOUS: "Using methocel cellulose ethers for controlled release of drugs in hydrophilic matrix systems", INTERNET CITATION, 1 July 2002 (2002-07-01), pages 1-36, XP002693777, Retrieved from the Internet: URL:http://www.colorcon.com/literature/mar keting/mr/Extended%20Release/METHOCEL/Engl ish/hydroph_matrix_broch.pdf [retrieved on 2013-03-14]

## Description

### FIELD OF THE INVENTION

This invention relates generally to low viscosity cellulosic rheology modifiers that are useful in hair care formulations. The low viscosity cellulosics exhibit a gelation temperature of more than 45°C.

### BACKGROUND

Hair care products must be effective at delivering active ingredients, such as styling polymers and conditioning polymers, while still having good aesthetic properties. From an end-user perspective, consumers associate good aesthetics, such as pleasant tactile feel and a pleasing visual appearance, with performance and value. For hair fixative compositions in particular, it is also desirable to that such compositions provide high volume and shine in hair, while leaving the hair with a light and natural feel.

To this end, many different types of rheology modifiers have been utilized in hair care compositions. For example, U.S. Patent Application Publication No. US 2014/0202485 discloses personal care compositions incorporating low gelation temperature methylcellulose imparting improved aesthetics. However, the prior art suffers from the disadvantage of requiring cooling to temperatures of 10°C or less in order to enable the methylcellulose to go into solution, which is necessary to provide a clear appearance and also obtain the desired performance benefit. Such required cooling, however, presents an obstacle to formulators when preparing an end-user composition.

WO 2013/048779 discloses a personal care compositions and methods incorporating low gelation temperature methylcellulose.

Consequently, there is a need to develop new additives for use in hair care formulations that provide high volume in hair in combination with a light and natural feel, while also eliminating the need to cool such components during preparation of an end-user composition.

### STATEMENT OF INVENTION

The present invention provides a hair care composition comprising
(a) a cellulose ether having (i) a methoxy substitution of from 21 to 42 weight % based upon the weight of the cellulose ether (ii) a viscosity of from 15 mPa-s to less than 4,000 mPa-s (15 cP to less than 4,000 cP) in a two percent aqueous solution at 20°C determined by Ubbelohde tube according to ASTM D1347-72 and D2363-79, and (iii) a gelation temperature of greater than 45°C measured as a 2% aqueous solution, and
(b) at least one hair fixative polymer, conditioning agent, or humectant,
wherein the cellulose ether is a hydroxypropyl methylcellulose further comprising a hydroxypropyl substitution of up to 32 weight % based upon the weight of the cellulose ether, and further wherein the hair care composition is selected from a spray, mousse, or gel

The specification further discloses a hair care composition comprising (a) a cellulose ether having a (i) a methoxy substitution of from 21 to 42 weight % based upon the weight of the cellulose ether (ii) a viscosity of from 15 cP to less than 4,000 cP in a two percent aqueous solution at 20°C, and (iii) a hydration temperature of 25°C or higher, and (b) at least one hair fixative polymer, conditioning agent, or humectant. This cellulose ether may also have a hydroxypropyl methylcellulose further comprising a hydroxypropyl substitution of up to 32 weight % based upon the weight of the cellulose ether.

The specification further discloses a method for inducing volume in hair comprising (1) applying to the hair a hair care composition comprising (a) a cellulose ether having (i) a methoxy substitution of from 21 to 42 weight % based upon the weight of the cellulose ether (ii) a viscosity of from 15 cP to less than 4,000 cP in a two percent aqueous solution at 20°C, and (iii) a gelation temperature of greater than 45°C, and (b) at least one hair fixative polymer, conditioning agent, or humectant; and (2) heat drying the hair. This cellulose ether may also have a hydroxypropyl methylcellulose further comprising a hydroxypropyl substitution of up to 32 weight % based upon the weight of the cellulose ether.

The specification further discloses a method for inducing volume in hair comprising (1) applying to the hair a hair care composition comprising (a) a cellulose ether having (i) a methoxy substitution of from 21 to 42 weight % based upon the weight of the cellulose ether, (ii) a viscosity of from 15 cP to less than 4,000 cP in a two percent aqueous solution at 20°C, and (iii) a hydration temperature of 25°C or higher, and (b) at least one hair fixative polymer, conditioning agent, or humectant; and (2) heat drying the hair. This cellulose ether may also have a hydroxypropyl methylcellulose further comprising a hydroxypropyl substitution of up to 32 weight % based upon the weight of the cellulose ether.

### BRIEF DESCRIPTION OF THE FIGURES

FIG. 1 shows the peak load (gmf) for hair tresses treated with inventive and comparative formulations using the Dia-Stron Wet Combing Method.
FIG. 2 shows the average percent increase in hair volume after application of inventive and comparative formulations to hair tresses using the Dia-Stron Wet Combing Method.

### DETAILED DESCRIPTION

The inventors have now surprisingly found that hydroxypropyl methyl cellulose (HPMC) are useful as additives in hair care compositions that provide a hair volumizing effect, while also eliminating the need to cool such components during preparation of an end-user composition. Accordingly, the present invention provides a hair care formulation comprising a cellulose ether having a methoxy substitution of from 21 to 42 weight % and a hydroxypropyl substitution of up to 32 weight %, based upon the weight of the cellulose ether, a viscosity of from 15 mPa-s to less than 4,000 mPa-s (15 cP to less than 4,000 cP) in a two percent aqueous solution at 20°C determined by Ubbelohde tube according to ASTM D1347-72 and D2363-79, and (iii) a gelation temperature of greater than 45 °C measured as a 2% aqueous solution; and at least one hair fixative polymer, conditioning agent, or humectant.

In the present invention, "hair care" relates to personal care compositions to be topically applied to a person (i.e., not ingested), and in particular a person's hair. Examples of hair care compositions include, but are not limited to, shampoos, leave-on conditioners, styling gels, hairsprays, and mousses. In certain preferred embodiments, the hair care composition is a mousse. Preferably, the hair care compositions are cosmetically acceptable. As used herein, "cosmetically acceptable" refers to ingredients typically used in personal care compositions, and is intended to underscore that materials that are toxic when present in the amounts typically found in personal care compositions are not contemplated as part of the present invention. The compositions of the invention may be manufactured by processes well known in the art, for example, by means of conventional mixing, dissolving, granulating, emulsifying, encapsulating, entrapping, or lyophilizing processes.

Cellulose has a polymeric backbone repeating structure of anhydroglucose units joined by 1-4 linkages. Each anhydroglucose unit contains hydroxyl groups at the 2, 3, and 6 positions. Substitution of these hydroxyls creates cellulose derivatives. For example, treatment of cellulosic fibers with caustic solution, followed by a methylating agent such as methyl chloride, yields cellulose ethers substituted with one or more methoxy groups. If not further substituted with other alkyls, this cellulose derivative is known as methyl cellulose (MC).

The cellulose ethers useful in the present invention are also substituted with one or more methoxy groups. The cellulose ethers used in the present invention contain both methoxy and hydroxypropyl substitutions. They are known as hydroxypropyl methylcellulose. In certain embodiments, the hydroxypropyl substituent group, -OCH₂CH(OH)CH₃, contains a secondary hydroxyl and may also be considered to form a propylene glycol ether of cellulose. The varying ratios of hydroxypropyl and methyl substitution is a factor which influences properties such as organic solubility and the thermal gelation temperature of aqueous solutions. In certain embodiments, the cellulose ethers useful in the present invention have a methoxy substitution of from 21 to 42 weight %, preferably of from 21 to 35 weight %, and more preferably of from 25 to 35 weight %, based upon the weight of the cellulose ether. In certain embodiments, the cellulose ethers useful in the present invention have a hydroxypropyl substitution of from 32 weight % or less, preferably of from 1 to 14 weight %, and more preferably of from 3 to 12 weight %, based upon the weight of the cellulose ether. The substitution is determined according to ASTM D2363-79. In certain embodiments, the cellulose ether polymers of the present invention have the following repeating structure:

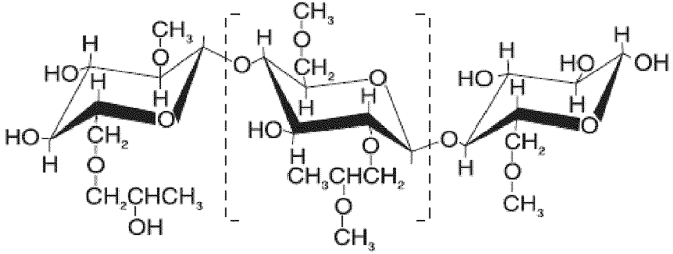

The difference in molecular weight of various methyl cellulose and hydroxypropyl methylcellulose polymers is reflected in the viscosity of an aqueous solution of a standard concentration. Viscosity of polymer solutions is the result of hydration of polymer chains, primarily through H-bonding of water molecules to the oxygen atoms in the numerous hydroxyl groups and ether linkages and the conformation rigidity of the anhydroglucose units in the polymer backbone, causing them to extend and form relatively open random coils. A given hydrated random coil is further H-bonded to additional water molecules, entrapping water molecules within, and may be entangled with other random coils. All of these factors contribute to larger effective size and increased frictional resistance to flow. As used herein, the term "viscosity" and the associated value for the 2% w/w aqueous solution is frequently used as a way to refer to the molecular weight of the polymer. The cellulose ethers useful in the present invention have a viscosity of from 15 mPa-s to less than 4,000 mPa-s (15 cP to less than 4,000 cP), preferably from 19 mPa-s to 1,500 mPa-s (19 cP to 1,500 cP), and more preferably from 19 mPa-s to 99 mPa-s (19 cP to 99 cP), in a two percent aqueous solution at 20°C. Viscosities of aqueous solutions are determined by Ubbelohde tube according to ASTM D1347-72 and D2363-79.

One unusual property of cellulose ethers of the present invention is that they exhibit reverse thermal gelation. In other words, aqueous solutions of HPMC will form gels at elevated temperature and give viscous solutions at cooler temperatures. Without wishing to be bound by theory, it is believed that thermal gelation temperature is governed by the nature and quantity of the substituent groups attached to the anhydroglucose ring and will thus vary according to the amount of methyl and hydroxypropyl substitution. Thus, hair care compositions comprising HPMC may be applied to hair followed by heating (e.g., blow-drying) to form a gel. The cellulose ethers useful in the present invention have a gelation temperature measured as a 2% aqueous solution of greater than 45°C, preferably from 58°C to 90°C, from 64°C to 70°C, or from 62°C to 68°C. In certain embodiments, the cellulose ether has a gelation temperature of from 58°C to 64°C. In certain embodiments, the cellulose ether has a gelation temperature of from 70°C to 90°C.

Methyl cellulose and hydroxypropyl methylcellulose dissolve in cold water by swelling and subsequent hydration. Without wishing to be bound by theory, it is believed that in a solution state at the hydration temperature, molecules are hydrated and there is little polymer-polymer interaction. As the temperature increases, the molecules gradually lose their water of hydration, and gelation will occur once a sufficient dehydration of the polymer occurs. Thus, the cellulose ether must be cooled to its hydration temperature in order to achieve a solution state in the inventive hair care formulations. In certain embodiments, the cellulose ethers useful in the present invention have a hydration temperature of 13°C or higher, 20°C or higher, 25°C or higher, or 29.5°C or higher.

Methods of making methyl cellulose and hydroxypropyl methylcellulose are described in detail in U.S. Patent No. 6,235,893. Generally, cellulose pulp is treated with a caustic solution, for example an alkali metal hydroxide, followed by an etherifying agent, e.g., methyl chloride (to achieve methoxy substitution) and/or propylene oxide (to achieve hydroxypropyl substitution). The resulting methyl cellulose or hydroxypropyl methylcellulose is washed to remove salt and other reaction by-products. Before or after washing, the cellulose ether may be stripped by exposure to steam to reduce residual organic content. The cellulose ether is then dried to a reduced moisture and volatile content of preferably 0.5 to 10.0 weight % water and more preferably 0.8 to 5.0 weight % water and volatiles based upon the weight of the HPMC. The reduced moisture and volatiles content enables the cellulose ether to be milled into particulate form. The cellulose ether is milled to particulates of desired size.

Once milled, aqueous solutions of HPMC may be prepared by dispersion technique. In certain embodiments, the milled HPMC is dispersed in heated water, and then cooled while agitating until hydration is complete. It is desirable to have adequate cooling after dispersion of the HPMC in hot water to ensure complete hydration. For improved clarity and reproducible control of viscosity, solutions of HPMC may require cooling in to a hydration temperature of from 13°C to 29.5°C, preferably of from 20°C to 25°C.

A person of ordinary skill in the art can readily determine the effective amount of HPMC that should be used in a particular composition in order to provide the desired benefits described herein (e.g., increased volume in hair) via a combination of general knowledge of the applicable field as well as routine experimentation where needed. By way of non-limiting example, the amount of HPMC in the hair care compositions of the invention may be in the range of from 0.01 to 10 weight %, preferably of from 0.05 to 5.0 weight %, and even more of from 0.1 to 1.0 weight %, based on the total weight of the composition.

In certain embodiments, the hair care compositions of the present invention include a hair fixative polymer. Suitable hair fixative polymers include, for example, PVP/VA copolymer, ethyl ester of PVM/MA copolymer, butyl ester of PVM/MA copolymer, vinyl acetate/crotonic acid copolymer, vinyl acetate/crotonic acid/vinyl neodecanoate, VA/butyl maleate/isobornyl acrylate copolymer, acrylates copolymer, diglycol/CHDM/isophthalates/SIP copolymer, acrylates/hydroxyester acrylates copolymer, methacrylates/acrylates copolymer/amine salt, AMP-acrylates/diacetone-acrylamide copolymer, AMPD-acrylates/diacetone-acrylamide copolymer, acrylates/methacrylate polymers, acrylates/acrylamide copolymer, PVP/vinyl caprolactam/DMAPA acrylates copolymer, polyvinylcaprolactam, isobutylene/ethylmaleimide/hydroxyethylmaleimide copolymer, acrylates/succinates/hydroxyacrylates copolymer, polyurethane-1, octylacrylamide/acrylates/butylaminoethyl methacrylate copolymer, vinyl caprolactam/VP/dimethylaminoethyl methacrylate copolymer, acrylates/t-butylacrylamide copolymer, acrylates/C1-2 succinates/hydroxyacrylates copolymer, acrylamide/sodium acryloyldimethyltaurate/acrylic acid polymer and mixtures thereof. The amount of hair fixative polymer effective for achieving the desired property provided by such components can be readily determined by one skilled in the art. In certain embodiments, the hair fixative polymer is present in a range of from 0.1 to 8.0 weight %, preferably of from 0.5 to 5.0 weight %, and more preferably of from 1.0 to 3.0 weight %.

In certain embodiments, the hair care compositions of the present invention include a conditioning agent. Suitable conditioning agents include, for example, quaternary ammonium salts of hydroxyethyl cellulose (e.g., Polyquaternium 67) commercially available from The Dow Chemical Company under the tradename SOFTCAT, polymeric quaternary ammonium salts of hydroxyethyl cellulose polymers (e.g., Polyquaternium 10) commercially available from The Dow Chemical Company under the tradename UCARE, and quaternary trialkylammonium halide compounds including, for example, dihydroxypropyl trialkyl ammonium chloride commercially available from The Down Chemical Company under the tradename PD QUAT. In certain embodiments, the conditioning agent is present in a range of from 0.1 to 20 weight %, preferably of from 0.5 to 10 weight %, and more preferably of from 1.0 to 3.0 weight %.

In certain embodiments, the hair care compositions of the present invention include a humectant to prevent loss of moisture. Suitable humectants include, for example, glycerin, sorbitol, monoglycerides, lecithins, glycolipids, fatty alcohols, fatty acids, polysaccharides, sorbitan esters, polysorbates (e.g., Polysorbate 20, Polysorbate 40, Polysorbate 60, and Polysorbate 80), diols (e.g., propylene glycol), diol analogs, triols, triol analogs, polymeric polyols, and mixtures thereof. In certain embodiments, the humectant is present in an amount of from 0.1 to 20 weight %, preferably 2 to 15 weight %, and more preferably 5 to 10 weight %, by weight of the hair care composition.

In certain embodiments, the hair care composition contains excipients, such as additional rheology modifiers/thickeners, emollients (e.g., hydrocarbon oils, esters, natural oils, or silicones), waxes, sensory modifiers, preservatives/antioxidants/chelating agents, reducing agents, pH adjusting agents/buffers/neutralizing agents, sunscreen actives, vitamins, proteins/amino acids, plant extracts, natural ingredients, bio-actives, fragrances/perfumes, foaming agents, penetrants, surfactants/detergents/emulsifiers/opacifying agents, volatiles/propellants/solvents/carriers, liquid vehicles/solvents/carriers, salts, anti-static agents, anti-frizz agents, antidandruff agents, hair waving/straightening agents, absorbents, colorants, hard particles, and other silicones. The amount of optional ingredients effective for achieving the desired property provided by such ingredients can be readily determined by one skilled in the art.

The hair care compositions of the present invention also include a dermatologically acceptable carrier. Such material is typically characterized as a carrier or a diluent that does not cause significant irritation to the skin and does not negate the activity and properties of active agent(s) in the composition. Examples of dermatologically acceptable carriers that are useful in the invention include, without limitation, water, such as deionized or distilled water, emulsions, such as oil-in-water or water-in-oil emulsions, alcohols, such as ethanol, isopropanol or the like, acetone, glycols, such as propylene glycol, glycerin or the like, creams, aqueous solutions, oils, ointments, pastes, gels, lotions, milks, foams, suspensions, powders, or mixtures thereof. In some embodiments, the composition contains from about 99.99 to about 50 percent by weight of the dermatologically acceptable carrier, based on the total weight of the composition.

As noted above, the hair care compositions of the present invention are highly effective as additives for providing a volumizing effect on hair because of the reverse thermal gelation properties of the hydroxypropyl methylcellulose included in the composition. HPMC according to the invention exhibits volumizing attributes on par with, if not better than, previously known additives for hair care applications, without the disadvantage of a low hydration temperature for purposes of dispersing the HPMC in the composition, thereby simplifying the formulation process for the hair care composition. Accordingly, the hair care compositions of the present invention are useful for the treatment of hair. Thus, in one aspect the present invention provides that the hair care compositions may be used in a method for styling hair.

In practicing the methods of the invention, the hair care compositions are administered topically by applying the compositions onto the hair via a spray, mousse, or gel. A person of ordinary skill in the art can readily determine the frequency with which the compositions should be applied. The frequency may depend, for example, on the level of humidity that an individual is likely to encounter in a given day and/or the sensitivity of the individual to low humidity. By way of non-limiting example, administration on a frequency of at least once per day may be desirable.

Some embodiments of the invention will now be described in detail in the following Examples.

### EXAMPLES

### Example 1

### Preparation of Exemplary Hair Care Formulations

Exemplary hair care compositions of the present invention contain the components recited in Table 1.

**Table 1. Exemplary Hair Care Compositions**

| **Phase** | **Component** | **INCI Name** | **% Solids** | **Final wt%** | **E1** | **E2** |
|---|---|---|---|---|---|---|
| A | DI water | DI water | -- | q.s. to 100 | 79.73 | 79.73 |
| A | METHOCEL E19* | Hydroxypropyl Methylcellulose | 2 | 0.25 | 12.50 | -- |
| A | METHOCEL K99** | Hydroxypropyl Methylcellulose | 2 | 0.25 | -- | 12.50 |
| A | AMP Ultra PC 2000 | Aminomethyl Propanol | 100 | 0.42 | 0.42 | 0.42 |
| A | Acudyne 1000 | Acrylates/Hydroxyesters Acrylates Copolymer | 45 | 1.80 | 4.00 | 4.00 |
| A | Aculyn 88 | Acrylates/Steareth-20 Methacrylate Crosspolymer | 29 | 0.58 | 2.00 | 2.00 |
| B | Propylene Glycol | Propylene Glycol | 100 | 0.20 | 0.20 | 0.20 |
| B | Tagat CH40 | PEG-40 Hydrogenated Castor Oil | 100 | 0.10 | 0.10 | 0.10 |
| B | Brij 30 | Polyoxyethylene (4) Lauryl Ether | 100 | 0.10 | 0.10 | 0.10 |
| B | Cocamidopropyl betaine | Cocamidopropyl Betaine | 36 | 0.18 | 0.50 | 0.50 |
| B | Neolone PE | Methylisothiazolinone and Phenoxyethanol | 100 | 0.45 | 0.45 | 0.45 |
| **Total** | | | 100 | 100 | 100 | 100 |

| | | | | | | |
|---|---|---|---|---|---|---|
| *METHOCEL E19 = Hydroxypropyl methylcellulose having a viscosity of 19 cP in a two percent aqueous solution at 20°C, a gelation temperature of from about 58-64°C, and a hydration temperature of about 25°C (available from The Dow Chemical Company) **METHOCEL K99 = Hydroxypropyl methylcellulose having a viscosity of 99 cP in a two percent aqueous solution at 20°C, a gelation temperature of from about 70-90°C, and a hydration temperature of about 29.5°C (available from The Dow Chemical Company) | | | | | | |

A 2% solids solution of each hydroxypropyl methylcellulose (i.e., METHOCEL E19 and METHOCEL K99) was prepared by slowly adding the respective HPMC to water heated to 80-90°C and stirring until well hydrated. The solution was then allowed to cool to room temperature. A mousse was then formulated by charging the 2% solids HPMC solution to water in a mixing vessel. The AMP Ultra PC 2000 was added with stirring. An emulsion polymer of Acudyne 1000 and Aculyn 88 was then added with stirring. The additional components in Phase B were added in order to complete the concentrate formulation. An aerosol can was then filled with 94% of the resulting concentrate solution, and then charged with butane (3%) and propane (3%).

### Example 2

### Preparation of Comparative Hair Care Formulations

Comparative hair care compositions of the present invention contain the components recited in Table 2.

**Table 2. Comparative Hair Care Compositions**

| **Phase** | **Component** | **INCI Name** | **% Solids** | **Final wt%** | **C1** | **C2** | **C3** |
|---|---|---|---|---|---|---|---|
| A | DI water | DI water | -- | q.s. to 100 | 92.23 | 79.73 | 79.73 |
| A | METHOCEL SG A7C⁺ | Methylcellulose | 2 | 0.25 | -- | 12.50 | -- |
| A | METHOCEL K4M⁺⁺ | Hydroxypropyl Methylcellulose | 2 | 0.25 | -- | -- | 12.50 |
| A | AMP Ultra PC 2000 | Aminomethyl Propanol | 100 | 0.42 | 0.42 | 0.42 | 0.42 |
| A | Acudyne 1000 | Acrylates/Hydroxyesters Acrylates Copolymer | 45 | 1.80 | 4.00 | 4.00 | 4.00 |
| A | Aculyn 88 | Acrylates/Steareth-20 Methacrylate Crosspolymer | 29 | 0.58 | 2.00 | 2.00 | 2.00 |
| B | Propylene Glycol | Propylene Glycol | 100 | 0.20 | 0.20 | 0.20 | 0.20 |
| B | Tagat CH40 | PEG-40 Hydrogenated Castor Oil | 100 | 0.10 | 0.10 | 0.10 | 0.10 |
| B | Brij 30 | Polyoxyethylene (4) Lauryl Ether | 100 | 0.10 | 0.10 | 0.10 | 0.10 |
| B | Cocamidopropyl betaine | Cocamidopropyl Betaine | 36 | 0.18 | 0.50 | 0.50 | 0.50 |
| B | Neolone PE | Methylisothiazolinone and Phenoxyethanol | 100 | 0.45 | 0.45 | 0.45 | 0.45 |
| **Total** | | | 100 | 100 | 100 | 100 | 100 |

| | | | | | | | |
|---|---|---|---|---|---|---|---|
| ⁺METHOCEL SG A7C = Methylcellulose having a viscosity of 700 cP in a two percent aqueous solution at 20°C, a gelation temperature of from about 38-44°C, and a hydration temperature of about 10°C (available from The Dow Chemical Company) ⁺⁺METHOCEL K4M = Hydroxypropyl methylcellulose having a viscosity of 4,000 cP in a two percent aqueous solution at 20°C, a gelation temperature of from about 70-90°C, and a hydration temperature of about 29.5°C (available from The Dow Chemical Company) | | | | | | | |

Comparative formulation C1 was a control base formulated without volumizing polymer. Comparative formulations C2 and C3 contained METHOCEL SG A7C and METHOCEL K4M, respectively. A 2% solids solution of METHOCEL SG A7C was prepared by slowly adding the methylcellulose to water heated to 80-90°C and stirring until well hydrated. The solution was then cooled in an ice bath to about 10°C while stirring. A 2% solids solution of METHOCEL K4M was prepared by slowly adding the hydroxypropyl methylcellulose to water heated to 80-90°C and stirring until well hydrated. The solution was then allowed to cool to room temperature while stirring. A mousse was then formulated by charging each of the 2% solids methylcellulose and HPMC solution to water in a mixing vessel. The AMP Ultra PC 2000 was added with stirring. An emulsion polymer of Acudyne 1000 and Aculyn 88 was then added with stirring. The additional components in Phase B were added in order to complete the concentrate formulation. An aerosol can was then filled with 94% of the resulting concentrate solution, and then charged with butane (3%) and propane (3%).

### Example 3

### Volume Enhancement Measurements

The formulations as prepared in Examples 1 and 2 above were evaluated for the enhancement of hair volume after application to hair. Sample hair tresses were washed with 10% Tergitol 15-S-9 and allowed to air dry overnight. Each test formulation (as prepared in Examples 1 and 2 above from E1, E2, C1, C2, and C3) was applied to three different sample tresses. Each tress of hair was evaluated three times to obtain an initial untreated volume measurement using the Dia-Stron Wet Combing Method: (i) ring size = 0.5 inches; (ii) start position = 20 mm; (iii) end position of 160 mm for 22.5 cm long hair, 145 mm for 20.5 cm medium hair, and 130 mm for 18.5 cm short hair; (iv) rate = 150 mm/min. Each tress was separately hydrated under lukewarm tap water for 20 seconds per side. Excess water was squeezed from the hair tresses using two fingers of a gloved hand. Each tress was then combed through twice with a wide tooth comb and twice with a fine tooth comb. After combing, 0.2 g (10% of hair weight) of each test mousse (as prepared in Examples 1 and 2 above from E1, E2, C1, C2, and C3) was dispensed on each hair tress and massaged into the hair with a gloved hand until all of the mousse appeared to be evenly distributed into the hair. Each hair tress was then hung from a clip or ring stand and dried using an Andis Professional 1800 hair dryer on highest and hottest settings while combing the hair with a 1.75 inch round brush. The temperature of the blow dryer was about 80-85°C at a distance of approximately 5 cm from the hair. The drying process was kept consistent by completing 3-4 pass-throughs of the hair with the brush and drying the hair completely in 1.5 minutes. The tresses were then allowed to equilibrate to room temperature in the control-temperature room for 1 hour. Post-treatment volume measurements were then collected from the hair using the same Dia-Stron Wet Combing Method described above.

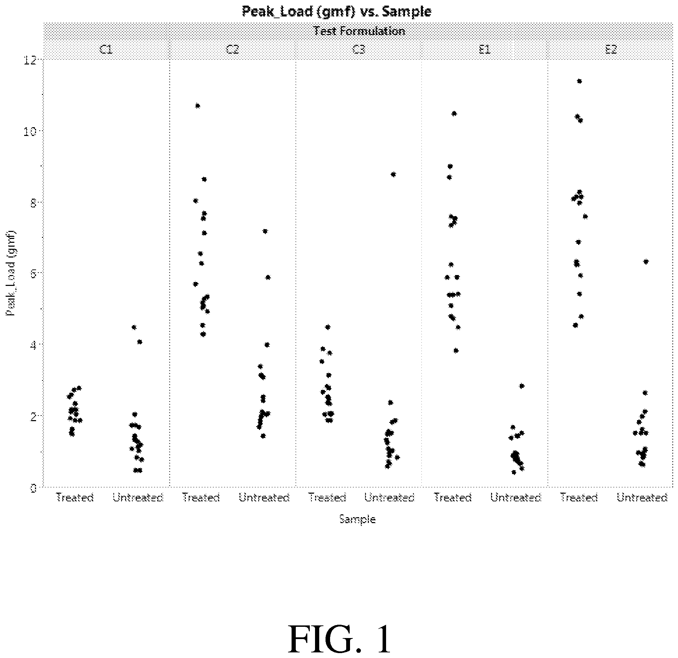

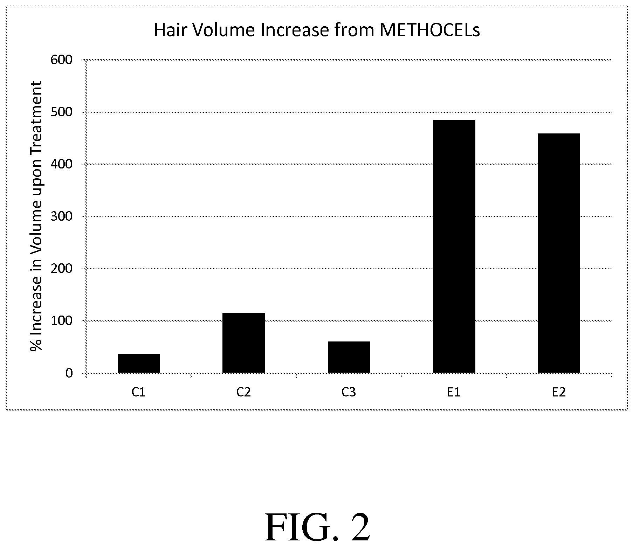

As shown in FIG. 1, hair tresses treated with inventive hydroxypropyl methylcellulose hair care compositions E1 and E2 demonstrate a significantly higher peak load (the max gmf at any given point along the tress being evaluated) than the comparative formulations. A higher peak load is an indication of more voluminous hair-the more force that is needed to pull the ring over the tress the higher volume that the hair retains. Similarly, FIG. 2 shows that hair tresses treated with inventive hydroxypropyl methylcellulose hair care compositions E1 and E2 demonstrated a significantly higher percent increase in hair volume (i.e., the average peak load of untreated hair verses treated hair) than the comparative formulations.

The above results demonstrate that the inventive hair care compositions containing hydroxypropyl methylcellulose provide superior hair volume performance when applied to hair as compared to formulations containing methylcellulose having a hydration temperature of less than 10°C and a gelation temperature of less than 45°C.

## Claims

1. A hair care composition comprising:
(a) a cellulose ether having (i) a methoxy substitution of from 21 to 42 weight % based upon the weight of the cellulose ether, (ii) a viscosity of from 15 mPa-s to less than 4,000 mPa-s (15 cP to less than 4,000 cP) in a two percent aqueous solution at 20°C determined by Ubbelohde tube according to ASTM D1347-72 and D2363-79, and (iii) a gelation temperature of greater than 45°C measured as a 2% aqueous solution; and
(b) at least one hair fixative polymer, conditioning agent, or humectant,
wherein the cellulose ether is a hydroxypropyl methylcellulose further comprising a hydroxypropyl substitution of up to 32 weight % based upon the weight of the cellulose ether, and further wherein the hair care composition is selected from a spray, mousse, or gel.

2. The hair care composition of claim 1, wherein the cellulose ether has a viscosity of from 19 mPa-s to less than 1,500 mPa-s (19 cP to 1,500 cP).

3. The hair care composition of claim 1, wherein the cellulose ether is present in the composition in a range of from 0.1 to 10 weight % by weight of the composition.

4. The hair care composition of claim 1, further comprising a propellant.

## Patentansprüche

1. Eine Haarpflegezusammensetzung, die Folgendes beinhaltet:
(a) einen Celluloseether mit (i) einer Methoxysubstitution von 21 bis 42 Gew.-%, bezogen auf das Gewicht des Celluloseethers, (ii) einer Viskosität von 15 mPa-s bis weniger als 4000 mPa-s (15 cP bis weniger als 4000 cP) in einer zweiprozentigen wässrigen Lösung bei 20 °C, bestimmt durch ein Ubbelohde-Rohr nach ASTM D1347-72 und D2363-79, und (iii) einer Gelierungstemperatur von mehr als 45 °C, gemessen als eine 2%ige wässrige Lösung; und
(b) mindestens ein Haarfestigerpolymer, ein Konditionierungsmittel oder ein Benetzungsmittel,
wobei der Celluloseether eine Hydroxypropylmethylcellulose ist, die ferner eine Hydroxypropylsubstitution von bis zu 32 Gew.-%, bezogen auf das Gewicht des Celluloseethers, beinhaltet, und ferner wobei die Haarpflegezusammensetzung aus einem Spray, einem Schaum oder einem Gel ausgewählt ist.

2. Haarpflegezusammensetzung gemäß Anspruch 1, wobei der Celluloseether eine Viskosität von 19 mPa-s bis weniger als 1500 mPa-s (19 cP bis 1500 cP) aufweist.

3. Haarpflegezusammensetzung gemäß Anspruch 1, wobei der Celluloseether in einem Bereich von 0,1 bis 10 Gew.-% nach Gewicht der Zusammensetzung in der Zusammensetzung vorhanden ist.

4. Haarpflegezusammensetzung gemäß Anspruch 1, die ferner ein Treibmittel beinhaltet.

## Revendications

1. Une composition pour le soin des cheveux comprenant :
(a) un éther de cellulose ayant (i) une substitution méthoxy allant de 21 à 42 % en poids rapporté au poids de l'éther de cellulose, (ii) une viscosité allant de 15 mPa-s à moins de 4 000 mPa-s (15 cP à moins de 4 000 cP) dans une solution aqueuse à deux pour cent à 20 °C déterminée par tube d'Ubbelohde selon l'ASTM D1347-72 et D2363-79, et (iii) une température de gélification de plus de 45 °C mesurée en solution aqueuse à 2 % ; et
(b) au moins un polymère fixateur de cheveux, un agent de conditionnement, ou un humectant,
où l'éther de cellulose est une hydroxypropyl méthylcellulose comprenant en sus une substitution hydroxypropyle allant jusqu'à 32 % en poids rapporté au poids de l'éther de cellulose, et en sus où la composition pour le soin des cheveux est sélectionnée parmi un spray, une mousse, ou un gel.

2. La composition pour le soin des cheveux de la revendication 1, où l'éther de cellulose a une viscosité allant de 19 mPa-s à moins de 1 500 mPa-s (19 cP à 1 500 cP).

3. La composition pour le soin des cheveux de la revendication 1, où l'éther de cellulose est présent dans la composition dans une gamme allant de 0,1 à 10 % en poids, en poids de la composition.

4. La composition pour le soin des cheveux de la revendication 1, comprenant en sus un propulseur.
